Europäisches Patentamt

European Patent Office

Office européen des brevets

(19)

(11) Veröffentlichungsnummer: **0 006 233**
**A1**

(12) ## EUROPÄISCHE PATENTANMELDUNG

(21) Anmeldenummer: 79101966.4

(22) Anmeldetag: 15.06.79

(51) Int. Cl.³: **A 61 K 7/32**

(30) Priorität: 19.06.78 DE 2626759

(71) Anmelder: Henkel Kommanditgesellschaft auf Aktien, Postfach 1100, D-4000 Düsseldorf 1 (DE)

(43) Veröffentlichungstag der Anmeldung: 09.01.80 Patentblatt 80/1

(72) Erfinder: Osberghaus, Rainer, Dr., Flösserstrasse 20, D-4000 Düsseldorf 13 (DE)

(84) Benannte Vertragsstaaten: CH GB NL SE

(54) Verwendung einer Kombination von Estern ein- und zweibasischer aliphatischer Hydroxycarbonsäuren mit Antioxidantien als Deodorantien.

(57) Gegenstand der Erfindung ist die Verwendung einer Kombination von Estern ein- und zweibasischer aliphatischer Hydroxycarbonsäuren mit 2-4 C-Atomen im Molekül mit aliphatischen oder alicyclischen Alkoholen mit 1-6 C-Atomen im Molekül, insbesondere in Gestalt der Neutralester, mit Antioxidantien in kosmetischen Mitteln als Deodorantien zur Unterdrückung von Körpergeruch.

EP 0 006 233 A1

4000 Düsseldorf, den 15.6.1978
Henkelstraße 67

HENKEL KGaA
ZR-FE/Patente
z-sü

P a t e n t a n m e l d u n g

D 5812

"Verwendung einer Kombination von Estern ein- und zweibasischer aliphatischer Hydroxycarbonsäuren mit Antioxidantien als Deodorantien"

---

Es ist bekannt, daß der störende Geruch, der die Perspiration des Menschen begleitet, durch die bakterielle
Zersetzung des zunächst geruchlosen Schweißes verursacht
wird. Um diesem Übelstand zu begegnen, wurden im wesentlichen 2 Wege eingeschlagen, einmal der Einsatz
von Verbindungen, die die Schweißabsonderung unterbinden. Daneben spielen noch rein sorptiv wirkende sowie
geruchsüberdeckende Mittel eine völlig untergeordnete
Rolle. Bei den kosmetischen Mitteln mit desodorierender
Wirkung handelt es sich im Gegensatz zu den Antiperspirantien durchweg um Mittel mit einem Gehalt an antimikrobiellen Stoffen. Als solche wurden z.B. Phenolderivate mit und ohne Halogensubstituenten, organische
Quecksilberverbindungen, quartäre Ammoniumverbindungen,
desinfizierend wirkende Abkömmlinge von Aminosäuren vorgeschlagen und zum Teil auch eingesetzt. Wenn bei dem
Einsatz der Deodorantien die Gefahr von Hautreizungen
nicht in so hohem Ausmaß wie bei der Verwendung von Antiperspirantien heraufbeschworen wird, so treten auch bei
der laufenden Benutzung von Antimikrobika enthaltenden
Deodorantien gelegentliche Unverträglichkeiten, Lichtsensibilisierungen und toxische Nebenwirkungen unterschiedlicher Stärke auf. Darüber hinaus ist die Mehrzahl

/2

dieser Produkte nicht geruchlos, viele besitzen einen leicht phenolischen Geruch. Den aufgezeigten Mängeln konnte durch den Gegenstand des Hauptpatentes, nämlich die Verwendung von Estern ein- und zweibasischer aliphatischer Hydroxycarbonsäuren mit 2 - 4 Kohlenstoffatomen im Molekül mit aliphatischen oder alicyclischen Alkoholen mit 1 - 6 Kohlenstoffatomen im Molekül weitgehend abgeholfen werden. Obwohl diese Ester keine schweißhemmenden bzw. antimikrobiellen Mittel im eigentlichen Sinne darstellen, ließen sich mit ihrer Hilfe dennoch sehr gut desodorierende, geruchsneutrale und von Nebenwirkungen weitgehend freie kosmetische Mittel herstellen.

In der deutschen Patentanmeldung P 24 18 362.8 wird die Verwendung von Estern ein- und zweibasischer aliphatischer Hydroxycarbonsäuren mit 2 - 4 Kohlenstoffatomen im Molekül mit aliphatischen oder alicyclischen Alkoholen mit 1 - 6 Kohlenstoffatomen im Molekül, insbesondere in Gestalt der Neutralester, als Deodorantien in wasserfreien kosmetischen Zubereitungen zur Unterdrückung von Körpergeruch, wobei die Ester in einer Menge von 1 - 25 Gewichtsprozent in der Zubereitung eingesetzt werden, beschrieben.

Es wurde nun gefunden, daß sich die desodorierende Wirkung
der Ester ein- und zweibasischer aliphatischer Hydroxycarbonsäuren mit 2 - 4 Kohlenstoffatomen im Molekül,
insbesondere deren Neutralestern, mit aliphatischen oder
alicyclischen Alkoholen mit 1 - 6 Kohlenstoffatomen im
Molekül erheblich steigern läßt, wenn man den desodorierenden kosmetischen Mitteln Antioxidantien zusetzt.

Die erfindungsgemäß einzusetzenden Ester der ein- und
zweibasischen aliphatischen 2 - 4 Kohlenstoffatome
im Molekül enthaltenden Hydroxycarbonsäuren lassen sich
in bekannter Weise durch azeotrope Veresterung der
Säuren mit dem jeweiligen Alkohol herstellen. Dabei
können als ein- und zweibasische aliphatische Hydroxycarbonsäuren zum Beispiel Glykolsäure, Milchsäure,
β-Hydroxypropionsäure, β-Hydroxybuttersäure, Glycerinsäure, Tartronsäure, Apfelsäure, Weinsäure eingesetzt
werden.

Als zu veresternde Alkohole kommen z.B. Methanol, Äthanol,
Propanol, Isopropanol, Butanol-1, Butanol-2, 2-Methylpropanol-1, 2-Methylpropanol-2, 2-Methylbutanol-1,
2-Methylbutanol-4, n-Hexylalkohol, Äthylenglykol,
Propylenglykol, Trimethylenglykol, Hexamethylenglykol, Glycerin, Erythrit, Sorbit und Cyclohexanol
in Frage.

Unter den erfindungsgemäß einzusetzenden Estern der
aliphatischen Hydroxycarbonsäuren kommt sowohl von der
desodorierenden Wirkung als auch von der anwendungstechnischen Eignung den jeweiligen neutralen Estern
die größte Bedeutung zu und unter diesen wiederum den
neutralen Estern mit aliphatischen einwertigen Alkoholen mit 1 - 6 Kohlenstoffatomen im Molekül.

/4

Als erfindungsgemäß einzusetzende Ester aliphatischer
Hydroxycarbonsäuren sind demnach zum Beispiel
Glykolsäurecyclohexylester, -glykolester, -glycerin-
ester, Milchsäurepropylenglykolester, -sorbitester,
-Oxybuttersäuremethylester, -isopropylester,
-hexylester, -erythritester, Glycerinsäurecyclohexylester, -tirmethylenglykolester, Tartronsäuremonomethylester, -monoäthylester, -monopropylester,
-monobutylester, monoamylester, -monohexylester,
Tartronsäureglykolester, -glycerinester, Äpfelsäuremonomethylester, -monoäthylester, -monoisopropyl-
ester, -monoisobutylester, -mono-tert.-butylester,
-monoamylester, Äpfelsäurecyclohexylester, -propylenglykolester, -hexamethylenglykolester, Weinsäuremonomethylester, -monoäthylester, -monopropylester,
-monoisopropylester, -monobutylester, -monoamylester,
-monohexylester, Weinsäureglykolester, -erythritester,
-sorbitester insbesondere jedoch Glykolsäuremethylester,
-äthylester, -propylester, -isopropylester, -butylester,
-isobutylester, -tert.-butylester, amylester, -hexylester,
Milchsäuremethylester, äthylester, -propylester,
-isopropylester, -butylester, -tert.butylester, -
-hexylester, β-Hydroxypropionsäuremethylester,
-äthylester, -isopropylester, -butylester, -amylester,
Glycerinsäuremethylester, -äthylester, -propylester,
-butylester, -hexylester, Tartronsäuredimethylester,
-diäthylester, -diisopropylester, -dibutylester,
-diamylester, Äpfelsäuredimethylester, -diäthylester,
-dipropylester, -diisopropylester, -dibutylester,
-dihexylester, Weinsäuredimethylester, -diäthylester,
-dipropylester, -diisopropylester, -dibutylester,
-di-tert.-butylester, -diamylester, -dihexylester
zu nennen.

Als erfindungsgemäß einzusetzende Antioxidantien sind alle auf dem pharmazeutischen, kosmetischen und Nahrungsmittel-Sektor gebräuchlichen Antioxidantien geeignet und es sind folgende Produkte zu nennen:

Butylhydroxyanisol, Butylhydroxytoluol, Guajakharz, Lecithin, Nordihydroguajaretsäure, Propylgallat, Octylgallat, Dodecylgallat, Tocopherole, Trihydroxybutyrophenon, Ascorbinsäure, Ascorbylpalmitat, Dilaurylthiodipropionat, Distearylthiodipropionat, Monopropylcitrat, Thiodipropionsäure und Citraconsäure. Besondere Bedeutung kommt dabei dem Butylhydroxyanisol und Butylhydroxytoluol zu. Für den erfindungsgemäßen Einsatz besonders geeignet hat sich ein Gemisch von 2- und 3-tert.Butyl-4-hydroxyanisol mit 2,6-Di.tert.-butyl-4-methylphenol (Butylhydroxytoluol) im Verhältnis von 1 :9 bis 9:1 erwiesen.

Die erfindungsgemäß zu verwendende Kombination von Estern ein- und zweibasischer aliphatischer Hydroxycarbonsäuren und aliphatischen oder alicyclischen Alkoholen mit Antioxidantien kann in alle üblicherweise für Deodorantien gebräuchliche Zubereitungen eingearbeitet werden wie Puder, Stifte, Roll-on und Sprays, wobei der Deo-Spray das bevorzugte Einsatzgebiet ist. Die Einarbeitung erfolgt in bekannter Weise durch einfaches Vermischen oder Lösen in den anderen Komponenten der Zubereitung wie Lösungsmitteln, Wachsen, Fettsubstanzen, Polyglykolen, Pudergrundstoffen. Die in die erfindungsgemäßen kosmetischen Mittel mit desodorierender Wirkung einzuarbeitenden Mengen an Estern ein- und zweibasischer aliphatischer Hydroxycarbonsäuren mit 2 - 4 Kohlenstoffatomen im Molekül mit aliphatischen oder alicyclischen Alkoholen mit 1 - 6 Kohlenstoffatomen im Molekül betragen 0,5 bis 20 Gewichtsprozent, vorzugsweise 2 - 10 Gewichtsprozent, bezogen auf das gesamte Mittel. Die

/6

einzuarbeitenden Mengen an Antioxidantien betragen
0,01 bis 5 Gewichtsprozent, vorzugsweise 0,1 bis 3
Gewichtsprozent, bezogen auf das gesamte Mittel.

Es wurde ferner gefunden, daß es nicht erforderlich ist,
daß die dem Deodorans zugrundeliegende Zubereitung
wasserfrei bzw. wasserarm ist, vielmehr kann diese
erhebliche Mengen Wasser enthalten und in Ausnahmefällen sogar eine wäßrige Lösung darstellen.

Es ist bereits aus der deutschen Offenlegungsschrift
2 358 121 bekannt, die bakterizide Wirkung von Deodorantien durch den Zusatz von Antioxidantien zu steigern.
Als bakterizid wirkendes Desodorierungsmittel ist z.B.
2,4,4'-Trichlor-2'-hydroxydiphenyläther, eine allgemein
bekannte antimikrobielle Verbindung genannt. Es war
daher überraschend, daß sich die desodorierende Wirkung
von Estern ein- und zweibasischer aliphatischer Hydroxycarbonsäuren mit 2 - 4 Kohlenstoffatomen im Molekül
mit aliphatischen oder alicyclischen Alkoholen mit
1 - 6 Kohlenstoffatomen im Molekül, die keine antimikrobiellen Verbindungen darstellen, durch den Zusatz
von Antioxidantien erheblich steigern läßt.

Die nachfolgenden Beispiele sollen den Gegenstand der
Erfindung näher erläutern, ohne ihn jedoch hierauf zu
beschränken.

### Beispiele

Für eine vergleichende Wirksamkeitsprüfung wurde ein Panel-Test durchgeführt. Dieser wurde so angelegt, daß in einer Testserie jeweils drei Produkte (A, B und C) vergleichend geprüft werden konnten. Zur Vorbereitung der Probanden wurde an 15 Personen je 1 Stück Seife, die keine antimikrobiellen Wirkstoffe enthielt, mit der Anweisung ausgegeben, während der gesamten Testzeit von 3 Wochen außer dieser Seife weder andere Seifen noch Desodorantien bzw. Antiperspirantien noch Parfüms zu benutzen. In der ersten Woche, die als Vorbereitungswoche diente, wurde eine unkontrollierte Wäsche mit der vorgeschriebenen Seife durchgeführt. In der darauffolgenden Woche begann der eigentliche Test: Morgens wurde eine von einer Aufsichtsperson kontrollierte Achselwäsche durchgeführt. Die Testprodukte wurden sodann bei je 5 Probanden nach folgendem Schema unter standardisierten Bedingungen auf die linke bzw. rechte Achsel appliziert:

| linke Achsel | rechte Achsel | |
| --- | --- | --- |
| Produkt A | Produkt B | 5 Probanden |
| Produkt A | Produkt C | 5 Probanden |
| Produkt B | Produkt C | 5 Probanden |

In der dritten Woche wurden die Applikationsseiten gewechselt. Nach Applikation der Testprodukte wurden unter den Achseln der Probanden Achselblätter mittels Gummibändern befestigt, die über einen Zeitraum von 7 Stunden getragen werden mußten. Zur Geruchsbeurteilung wurden die Achselblätter nach Abschluß der Testzeit (7 Stunden) abgenommen, in kodierte, verschließbare und auf 37°C erwärmte Glasbehälter gegeben und die verschraubten Gläser anschließend 15 Minuten bei 37°C

/8

gelagert. Die Geruchsbeurteilung des links-rechts-Unterschiedes der Achselblätter jeder Versuchsperson (= 1 Prüfobjektpaar) erfolgte durch 3 trainierte Geruchsprüfer, die ihre Bewertung unabhängig voneinander abgaben. Jeder Prüfer erhielt für jeden Testtag ein neues Prüfformular und erstellte die Beurteilung nach folgendem Schema:

stärkerer Geruch      = Note 1
schwächerer Geruch  = Note 0
gleicher Geruch        = Note 0,5 für beide Proben.

Der Testleiter stellte sodann die Summe der Benotungen der Prüfobjektpaare zusammen:

| | $\Sigma$ der Bewertung der Prüfobjektpaare |
|---|---|
| A : B | $x$ Punkte : $y$ Punkte |
| A : C | $x'$ Punkte : $y'$ Punkte |
| B : C | $x''$ Punkte : $y''$ Punkte |

Durch Addition der Noten, die ein Produkt auf sich vereinigt, erhält man eine Rangfolge, bei welcher die jeweils höhere Gesamtnotensumme die schlechtere Wirkung des Produktes angibt:

| Produkt | Rangfolge |
|---|---|
| A | $x + x'$ Punkte |
| B | $y + x''$ Punkte |
| C | $y' + y''$ Punkte |

Der Montag als jeweils erster Applikationstag der Produkte blieb bei der Auswertung in beiden Testwochen unberücksichtigt.

/9

Aus den nachstehend aufgeführten Tabellen ist die gute Wirksamkeit der erfindungsgemäßen Kombinationen, die nicht auf einem rein additiven Effekt beruht, ersichtlich.

In den nachstehenden Versuchen haben die Kurzbezeichnungen folgende Bedeutung:

BHA = Stellungsisomerengemisch aus 2- und 3-tert.- Butyl-4-hydroxyanisol

BHT = 2,6-Di-tert.-butyl-4-methylphenol

Treibgas 11 = Monofluor-trichlor-methan

Treibgas 12 = Difluor-dichlor-methan

Treibgas 114 = 1,2-Tetrafluordichloräthan

Bei der beschriebenen Bewertung liegt ein signifikanter Unterschied auf Basis von mindestens 95 % statistischer Sicherheit bei einer Punktedifferenz von ≥ 19 Punkten vor.

<u>Versuch 1</u>

| <u>Spray A</u> | 3,0  | % Äpfelsäurediäthylester |
|---|---|---|
| | 0,1  | % BHA |
| | 0,1  | % BHT |
| | 26,8 | % Äthanol |
| | 70,0 | % Treibgas 11/12 40:60 |

| <u>Spray B</u> | 0,1  | % BHA |
|---|---|---|
| | 0,1  | % BHT |
| | 29,8 | % Äthanol |
| | 70,0 | % Treibgas (wie A) |

| <u>Spray C</u> | 3,0  | % Äpfelsäurediäthylester |
|---|---|---|
| | 27,0 | % Äthanol |
| | 70,0 | % Treibgas (wie A) |

| Auswertung | Punkte | Desodorierende Wirkung | Punktedifferenz |
|---|---|---|---|
| Spray A | 93,5 | A > B | 59,5 |
|  |  | A > C | 20,0 |
| Spray B | 153,0 |  |  |
| Spray C | 113,5 | C -> B | 39,5 |

## Versuch 2

Spray A          0,05 % DL-α-Tocopherol

5,0 % Capryl-/Caprinsäuretriglycerid

ad 100,0 % Treibgas 11/12 40:60

Spray B          0,05 % DL-α-Tocopherol

5,0 % Weinsäuredimethylester

ad 100,0 % Treibgas (wie A)

Spray C          5,0 % Weinsäuredimethylester

ad 100,0 % Treibgas (wie A)

| Auswertung | Punkte | Desodorierende Wirkung | Punktedifferenz |
|---|---|---|---|
| Spray A | 142,0 |  |  |
| Spray B | 99,5 | B > A | 42,5 |
|  |  | B >- C | 19,0 |
| Spray C | 118,5 | C > A | 23,5 |

## Versuch 3

Spray A      1,5  % Äpfelsäurediisopropylester
             0,5  % Weinsäurediäthylester
             0,2  % BHT : BHA = 1 : 9
            27,8  % Äthanol
            70,0  % Treibgas 11/12 40:60

Spray B      0,4  % BHT : BHA = 9 : 1
             2,6  % Isopropylmyristat
            27,0  % Äthanol
            70,0  % Treibgas (wie A)

Spray C      1,5  % Äpfelsäurediisopropylester
             0,5  % Weinsäurediäthylester
             0,2  % BHT : BHA = 9 : 1
            27,8  % Äthanol
      ad 100,0  % Treibgas (wie A)

| Auswertung: | Punkte | Desodorierende Wirkung | Punktedifferenz |
|---|---|---|---|
| Spray A | 107,5 | A > B | 37,0 |
|  |  | A = C | 0,5 |
| Spray B | 144,5 |  |  |
| Spray C | 108,0 | C > B | 36,5 |

/12

0006233

Die erfindungsgemäße Verwendung einer Kombination der Ester ein- und zweibasischer aliphatischer Hydroxycarbonsäuren mit 2 - 4 Kohlenstoffatomen im Molekül und aliphatischen oder alicyclischen Alkoholen mit 1 - 6 Kohlenstoffatomen im Molekül mit Antioxidantien kann z.B. in folgenden kosmetischen Mitteln mit desodorierender Wirkung erfolgen:

<u>Desodorierender Stift</u>

| | | |
|---|---|---|
| 2-Octyldodecanol | 26,5 | Gew.-Teile |
| Cetylstearylalkohol | 3,0 | " |
| Natriumstearat | 8,0 | " |
| Kokosfettsäuremonoäthanolamid | 3,0 | " |
| Paraffinöl | 2,0 | " |
| Propylenglykol | 2,5 | " |
| Äthanol | 49,5 | " |
| Weinsäuredibutylester | 5,0 | " |
| BHA + BHT 1:1 | 0,5 | " |

<u>Desodorierender Puder</u>

| | | |
|---|---|---|
| Reisstärke Derivat NAL R-5 | 12,0 | Gew.-Teile |
| Magnesiumcarbonat | 2,0 | " |
| Zinkoxid | 2,0 | " |
| Talkum extra fein | 76,0 | " |
| Äpfelsäuredihexylester | 7,5 | " |
| α-Tocopherol | 0,5 | " |

<u>Desodorierender Spray</u>

| | | |
|---|---|---|
| Milchsäurebutylester | 6,0 | Gew.-Teile |
| BHA | 0,5 | " |
| Äthanol | 29,5 | " |
| Isopropanol | 2,8 | " |
| Propylenglykol | 1,2 | " |
| Treibgas 12/114 60:40 | 60,0 | " |

Desodorierender Spray
Weinsäurediäthylester                    4,0 Gew.-Teile
BHT                                      0,5    "
Äthanol                                 10,0    "
Isopropanol                             18,0    "
Isopropylmyristat                        2,0    "
Treibgas 12/114 60:40                   65,0    "

Desodorierender Spray
Äpfelsäurediäthylester                   5,0 Gew.-Teile
BHA + BHT 1:1                            0,5    "
Capryl/Caprinsäuretriglycerid            4,0    "
Treibgas 12/114 60:40.                  90,5    "

Desodorierender Spray
Glykolsäurebutylester                   10,0 Gew.-Teile
α-Tocopherol                            0,5    "
Propylenglykol                           1,5    "
Isopropylstearat                         2,0    "
Treibgas 12/114 60:40                   86,0    "

Desodorierender Spray
Tartronsäurediisopropylester             7,5 Gew.-Teile
BHA + BHT 1:1                            0,5    "
Propylenglykol                           2,0    "
Isopropylmyristat                        2,0    "
Äthanol                                 13,0    "
Treibgas 11/12 50:50                    75,0    "

Desodorierender Spray
Glycerinsäurecyclohexylester             7,5 Gew.-Teile
BHA                                      0,5    "
Äthanol                                 29,0    "
Isopropylmyristat                        3,0    "
Treibgas 12                             60,0    "

0006233

### Desodorierender Spray

| | |
|---|---|
| Äpfelsäurediisopropylester | 3,8 Gew.-Teile |
| α-Tocopherol | 0,2 " |
| Äthanol | 27,0 " |
| Isopropanol | 7,0 " |
| Propylenglykol | 2,0 " |
| Treibgas 12/114 60:40 | 60,0 " |

### Desodorierender Spray

| | |
|---|---|
| Weinsäuredimethylester | 6,5 Gew.-Teile |
| BHA + BHT 1:1 | 0,5 " |
| Isopropylmyristat | 3,0 " |
| Äthanol | 20,0 " |
| Isopropanol | 10,0 " |
| Treibgas 11/12 50:50 | 60,0 " |

### Deo-Roll-on

| | |
|---|---|
| Äthanol | 45,0 Gew.-Teile |
| Äpfelsäurediäthylester | 1,0 " |
| BHA + BHT 1:4 | 0,2 " |
| Acrylsäurepolymerisat der Fa. Goodrich Chem. Corp. Carbopol 940$^{(R)}$ | 0,2 " |
| Triäthanolamin | 0,1 " |
| Wasser | 49,5 " |
| Nonylphenolpolyglykoläther der Elektrochem. Fabr. Kempen Merpoxen NO$^{(R)}$ | 4,0 " |

/15

0006233

Deodorant-Creme

| | |
|---|---|
| Gemisch von Mono- und Di-glyceriden der Palmitin- und Stearinsäure Cutina MD[(R)] Dehydag | 18,0 Gew.-Teile |
| Cetyl/Stearylalkohol + ca. 12 Mol Äthylenoxid Eumulgin B 1[(R)] Dehydag | 4,0 " |
| Ölsäuredecylester | 10,0 " |
| Weinsäuredimethylester | 5,0 " |
| BHA + BHT 1:9 | 0,5 " |
| Wasser | 61,4 " |
| p-Hydroxybenzoesäuremethylester | 0,1 " |
| Parfümöl | 1,0 " |

Deodorans für Pumpzerstäuber

| | |
|---|---|
| Äthanol | 81,0 Gew.-Teile |
| Isopropanol | 4,0 " |
| Äpfelsäurediisiopropylester | 1,0 " |
| BHA + BHT 9:1 | 0,3 " |
| Parfüm | 1,0 " |
| Wasser | 12,7 " |

Deodorans für Pumpzerstäuber

| | |
|---|---|
| Äthanol | 50,0 Gew.-Teile |
| Weinsäurediäthylester | 1,5 " |
| BHA + BHT 5:1 | 0,1 " |
| Parfüm | 1,0 " |
| Wasser | 47,4 " |

Patentanmeldung D 5812          1                    HENKEL KGaA
                                                     ZR-FE/Patente

"Verwendung einer Kombination von Estern ein- und
zweibasischer aliphatischer Hydroxycarbonsäuren
mit Antioxidantien als Deodorantien"

Patentansprüche:

1. Verwendung von Estern ein- und zweibasischer aliphatischer Hydroxycarbonsäuren mit 2 - 4 Kohlenstoffatomen im Molekül mit aliphatischen oder alicyclischen Alkoholen mit 1 - 6 Kohlenstoffatomen im Molekül, insbesondere in Gestalt der Neutralester in wasserfreien kosmetischen Zubereitungen in einer Menge von 1 - 25 Gewichtsprozent, bezogen auf die gesamte kosmetische Zubereitung, zur Unterdrückung von Körpergeruch, dadurch gekennzeichnet, daß die Ester der ein- und zweibasischen aliphatischen Hydroxycarbonsäuren in einer Menge von 0,5 - 20 Gewichtsprozent in Kombination mit Antioxidantien in einer Menge von 0,01 bis 5 Gewichtsprozent, beide jeweils bezogen auf die gesamte Zubereitung, verwendet werden.

2. Verwendung von Estern ein- und zweibasischer aliphatischer Hydroxycarbonsäuren in Kombination mit Antioxidantien nach Anspruch 1, dadurch gekennzeichnet, daß die Ester der ein- und zweibasischen aliphatischen Hydroxycarbonsäuren in einer Menge von 1 - 10 Gewichtsprozent und die Antioxidantien in einer Menge von 0,1 - 3 Gewichtsprozent, jeweils bezogen auf die gesamte Zubereitung, verwendet werden.

/2

3. Verwendung von Estern ein- und zweibasischer aliphatischer Hydroxycarbonsäuren in Kombination mit Antioxidantien nach Anspruch 1 und 2, dadurch gekennzeichnet, daß die Verwendung sowohl in wasserfreien wie auch wasserhaltigen kosmetischen Zubereitungen erfolgt.

4. Verwendung von Estern ein- und zweibasischer aliphatischer Hydroxycarbonsäuren in Kombination mit Antioxidantien nach Anspruch 1 - 3, dadurch gekennzeichnet, daß als Antioxidantien ein Gemisch von 2- und 3-tert.-Butyl-4-hydroxyanisol mit 2,6-Di-tert.butyl-4-methylphenol im Verhältnis 1:9 bis 9:1 eingesetzt wird.

0006233

**Europäisches Patentamt**

**EUROPÄISCHER RECHERCHENBERICHT**

Nummer der Anmeldung

**EP 79 10 1966**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 456 639 (H. SCHWARZKOPF) <br> * Ansprüche; Seite 3, Abschnitt 2; Seite 5, Abschnitt 2; Beispiele 1,2,9,12,14 * <br> -- | 1-4 |
| DA | DE - A - 2 418 362 (HENKEL) <br> * Ansprüche; Seite 2, Abschnitt 2 - Seite 5, Abschnitt 1 * <br> -- | 1 |
| A | FR - A - 2 192 798 (GILLETTE) <br> * Ansprüche * <br> -- | 1 |
| A | US - A - 3 098 795 (S.I. KREPS) <br> * Ansprüche; Spalte 4, Zeile 66 - Spalte 5, Zeile 20 * <br> -- | 1 |
| DA | DE - A - 2 358 121 (BEECHAM) <br> * Ansprüche; Seite 2, Abschnitt 6 - Seite 3, Abschnitt 1 * <br> -- | 1 |
| A | FR - A - 2 160 898 (THE MENNEN CY.) <br> * Ansprüche * <br> -- | 1 |
| A | FR - A - 2 099 582 (L'OREAL) <br> * Ansprüche, Seite 2, Zeilen 10-12; Beispiele * <br> ---- | 1 |

Der vorliegende Recherchenbericht wurde für alle Patentansprüche erstellt.

**KLASSIFIKATION DER ANMELDUNG (Int.Cl.²)**

A 61 K 7/32

**RECHERCHIERTE SACHGEBIETE (Int. Cl.²)**

A 61 K 7/32

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 07-09-1979 | VANHECKE |

EPA form 1503.1  06.78